# EUROPEAN PATENT SPECIFICATION

(11) **EP 0 926 233 B1**
(45) Date of publication and mention of the grant of the patent: **22.12.2004**
(21) Application number: 98119429.3
(22) Date of filing: 15.10.1998
(51) Int. Cl.: C12M 1/20, C12M 1/16

(54) **Device for counting and isolating microorganisms**
Vorrichtung zum Zählen und Isolieren von Mikroorganismen
Appareil pour le comptage et l'isolation de microorganismes

(30) Priority: 17.10.1997 IT CA970029
(43) Date of publication of application: 30.06.1999
(73) Proprietor: Microbiol S.n.c., 09010 Uta (Cagliari) (IT)
(72) Inventor: Murgia, Sergio, 09100 Cagliari (IT)
(74) Representative: Luppi, Luigi

(56) References cited:
- WO-A-91/00903
- DE-A- 2 805 995

## Description

The invention refers to the field of diagnostic devices for isolating and counting microorganisms in a culture medium.

DE-A-2805995 discloses a device comprising two chambers, separated by barrier means. A plurality of walls enables each chamber to be divided into a plurality of parallel channels. Only one of the opposite ends of each wall is joined to the walls of chamber means, in order to make channels each other communicating at the other ends and thus allow a molten culture medium containing micro-organisms to be poured into parallel channels by means of a single dispensing operation. This device serves to for test micro-organism against various substances which may act as promoting or inhibiting factors towards such micro-organisms, said substances being dispensed in each channel through suitable openings made in the body of the device itself. Therefore, it is possible to compare different effects produced by different substances, or different concentrations of the same substance, on a microbial strain by means of comparing microbial growing fronts in adjacent channels.

WO-A-91/0093 discloses a device which, in one of the embodiments, comprises a plurality of test regions in form of parallel channels, separated by walls and each other communicating by means of common regions. This structure enable the channels to be full with a culture medium containing micro-organisms by means of a single dispensing operation. Moreover, each test region is provided with a well occupying an intermediate portion of the channel and disposed to be charged with a substance to be tested towards micro-organisms, and a graduated scale. During incubation, micro-organisms grow in each channel, and test substances diffuse from wells along the channel, in opposite directions. The effects of test substances on micro-organisms are expressed by growth inhibition zones which form at opposite sides of each well. Reference marks comprised in scales enable an operator to match differently extended areas of growth inhibition with, for instance, different concentrations of a same test substance.

A disadvantage of DE-A-2805995 and WO-A-91/0093 is that while the effect of substances against micro-organisms is tested, no means are envisaged to achieve counting of micro-organisms.

The prior art comprises the italian patent application CA95A000007, filed on 08/06/1995, which discloses a device for use in diagnostics comprising a plurality of rectilinear grooves in which culture mediums are contained. One of said grooves has a greater length than the other grooves in order to allow the bacterial charges to be counted by means of a graduated scale printed on a side of said groove.

In use, a liquid specimen to be analysed is spread on the culture medium using a loop which is grazed on the medium along a rectilinear path.

This system allows the bacterial charges to be counted quickly, but shows, in some operative conditions, a limitation consisting of a limited capacity of isolating bacterial colonies in the specimen. In fact, when the specimen contains polymicrobial bacterial colonies with more than 10⁶ microorganisms per millilitre of specimen, the respective colonies have a confluent growth which makes substantially impossible to isolate them.

Therefore, a problem arise of improving the diagnostic devices of the type mentioned above, particularly in order to improve the isolation of bacterial charges present in the specimen.

According to the invention, a device for use in diagnostics is provided as defined in the claims.

Owing to the invention, it is possible for the well means to be so shaped as to extend through a considerable length without interruption: all that helps a regular distribution of the specimen through the culture medium and an optimum isolation of the bacterial colonies.

In an advantageous embodiment, the well means shows a "U" lay-out in plan view, which allows the length of the well means and of the culture medium to be doubled, without increasing the overall dimensions of the device.

In a further advantageous embodiment, the well means shows a "L" shaped lay-out in plan view, particularly when the container has a rectangular shape in plan view, said lay-out extending in a parallel direction of, and in close proximity to, one of the longer of sides and one of the shorter sides of the container converging in a same vertex.

This makes possible an optimum use of the space available in the container, because it is possible to use the areas of the container close to the side edges having a shorter length, which, otherwise, would be partly unusable.

Due to the invention, it is possible to perform the bacterial isolation and identification of the microbial charges contained in a liquid specimen.

In addition, due to the increased length of the well means in comparison with traditional containers, it is possible to count the charges even when there are more than 10⁶ microorganisms per millilitre of liquid specimen, which requires, however, that an appropriate measuring scale is identified.

According to a further aspect of the present invention, a device for use in diagnostics is provided, comprising a container equipped with well means having an elongated shape and associated with a graduated scale having a starting-point at an end of said well means and capable of showing the concentration of bacterial colonies present in the specimen, said scale having a reference mark placed at a distance of about 12,5 cm far from said starting point.

The reference mark mentioned above corresponds to a concentration of 10⁷ microorganisms per millilitre of liquid specimen and is placed at a distance of about 12,5 cm far from the left edge of the well.

The invention will be better understood and put into practice with reference to the attached drawings, which illustrate some exemplary embodiments of the invention, in which:

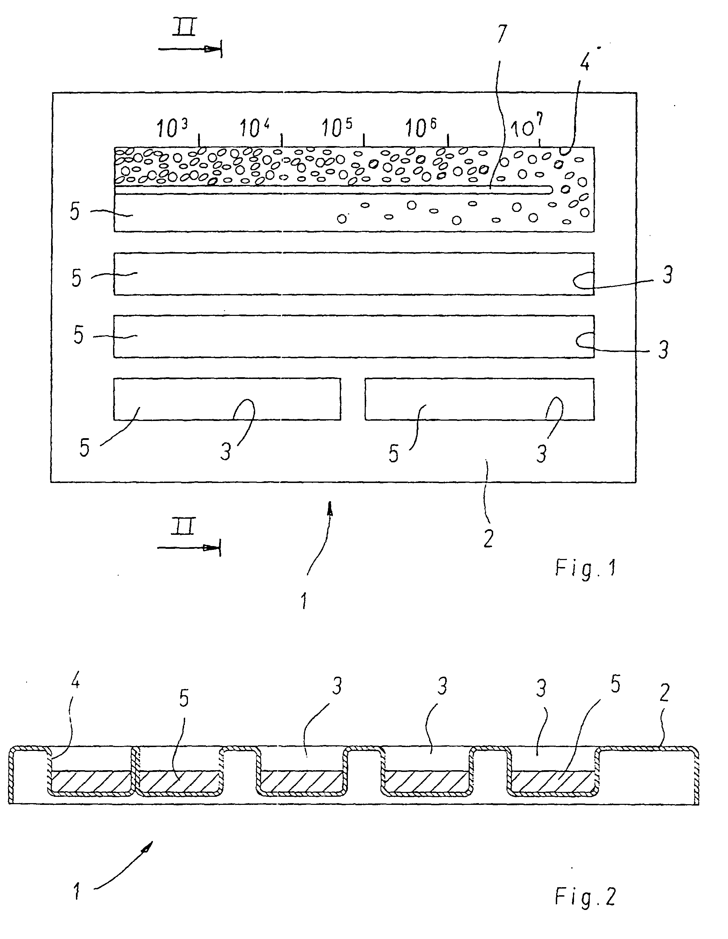

Figure 1 is a plan view of a device for use in diagnostics;
Figure 2 is a section through a plane II-II in figure 1;
Figure 3 is a plan view of a variation of the diagnostic device.

A diagnostic device 1 comprises a container body 2 of plastic material in which rectilinear cavities 3 having a rectangular shape in plan view are made by thermoforming. In addition, the container 2 is provided with a non-rectilinear cavity 4, having a "U" shaped lay-out in plan view, with branches rather close and parallel to each other. A culture medium 5 is contained in the cavities 3 and 4, said culture medium being preferably a generic culture medium for the "U" shaped cavities 4 and a selective culture medium for the rectilinear cavities 3.

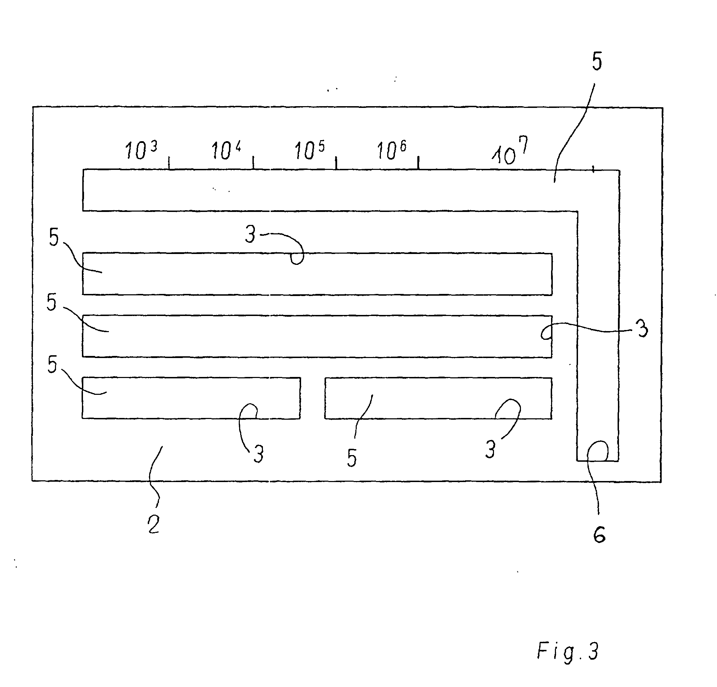

In the embodiment of figure 3, the container 2, in addition to the rectilinear cavities 3, is provided with a cavity 6, in which the culture medium 5 is inserted, said cavity having a "L" shaped lay-out and being disposed parallel to sides of the container converging in a same vertex.

When the device is used, the spreading of the specimen is obtained by grazing on the culture medium 5 a loop, which has been previously dipped in the liquid specimen to be analysed, through a linear path extending from an end of the cavity 4, or 6, towards the other end.

In such a way, quantities of the specimen, progressively decreasing from the position where the spreading begins to the position when the spreading ends, are distributed on the culture medium.

The spreading is preferably made without lifting the loop between said positions.

However, when using the version of the device provided with a "U" shaped cavity 4, it is possible to perform the spreading with two spreading paths, starting with a first spreading path beginning in the portion of the cavity comprising the stretch closer to the outer edge of the container 2 and continuing with a second spreading path, after lifting the loop from the contact with the culture medium, starting again the spreading in the other branch of the "U" beginning from the end thereof corresponding to the end at which the first path had started.

In this way, in the last portion of the spreading path, the loop has almost completely released the quantity of the liquid specimen that was carried at the beginning of the path, so that the distribution of the specimen on the culture medium is much more rarefied. Thus, the microbial colonies 7 are isolated from each other in the area farthest from the area at which the spreading was started. In this way, it is possible to isolate the bacterial charges, even with respect to single microbial families.

The fact that a single elongated cavity 4, or 6, has been provided makes possible to guarantee that a uniform and homogeneous composition and degree of filling of the cavity may be obtained, when the culture medium is introduced in the cavity in a liquid state, during the manufacture of the device.

The elongated, non-rectilinear, cavities 4 and 6 are preferably provided with a graduated scale for a counting of the colonies, said scale being applied by printing or in relief near a side of the cavities.

The reference marks concerning concentrations up to 10⁶ microorganisms per millilitre of liquid specimen are distributed as disclosed in the italian patent application CA95A000007, whilst the reference mark concerning the concentration of 10⁷ microorganisms per millilitre of liquid specimen is placed at a distance of 12,5 cm far from the left edge of the branch of the well 4, or 6, closer to the upper edge of the container 2.

## Claims

1. A device for counting and isolating microorganisms in a liquid sample, comprising a container (2) provided with elongated well means (3, 4) containing a culture medium (5) onto which said sample is spreadable along a path which continuously extends from a start position at one end of said well means (3, 4) to an end position of said well means (3, 4), **characterized in that** said well means (4) shows a "U"- shaped lay-out in plan view.

2. A device for counting and isolating microorganisms in a liquid sample, comprising a container (2) provided with elongated well means (3, 6) containing a culture medium (5) onto which said sample is spreadable along a path which continuously extend from a start position at one end of said well means (3, 6) to an end position of said well means (3, 6), **characterized in that** said well means (4) shows a "L"- shaped lay-out in plan view.

3. A device according to claim 1, or 2, and further comprising a graduated scale associated to said well means (3, 4; 3, 6), said scale being arranged for counting any microbial colonies (7) growing onto said culture medium (5) by comparing the position of said colonies (7) with reference marks of said scale.

4. A device according to claim 3, wherein said scale further comprises a reference mark placed at a distance of about 12, 5 cm far from a start point of said path.

5. A method for counting and isolating microorganisms in a liquid sample, comprising:
- dipping a loop in a liquid sample thereby trapping part of said sample in said loop;
- spreading said part onto a culture medium (5) contained in well means (3, 4; 3, 6) of a device according to claim 3 or 4;
**characterized by**
- performing said spreading along a non-rectilinear path which continuously extends from a start position at one end of said well means (3, 4; 3, 6) to an end position of said well means (3, 4; 3, 6);
- allowing microorganisms, if any, present in said sample to grow onto said culture medium (5) forming microbial colonies (7) which are isolated from each other;
- counting said colonies (7) by comparing the positions thereof onto said culture medium (5) with reference marks of a graduated scale associated with said well means (3, 4; 3, 6).

6. Use of a device as claimed in any of claims 1 to 4 for isolating and counting microorganisms.

## Patentansprüche

1. Vorrichtung zur Zählung und Isolierung von Mikroorganismen in einer flüssigen Probe, mit einem Behälter (2), welcher mit länglichen Well-artigen Mitteln (3, 4) versehen ist, welche ein Kulturmedium (5) enthalten, auf welchem die Probe entlang einer Strecke ausstreichbar ist, die sich kontinuierlich von einem Ausgangspunkt an einem Ende der länglichen Well-artigen Mittel (3, 4) zu einem Endpunkt der länglichen Well-artigen Mittel (3, 4) erstreckt, **dadurch gekennzeichnet, dass** das längliche Well-artige Mittel (4) in Draufsicht eine "U"-förmige Ausbildung zeigt.

2. Vorrichtung zur Zählung und Isolierung von Mikroorganismen in einer flüssigen Probe, mit einem Behälter (2), der mit länglichen Well-artigen Mitteln (3, 6) versehen ist, welche ein Kulturmedium (5) enthalten, auf welchem die Probe entlang einer Strecke ausstreichbar ist, die sich kontinuierlich von einem Ausgangspunkt an einem Ende der länglichen Well-artigen Mittel (3, 6) zu einem Endpunkt der länglichen Well-artigen Mittel (3, 6) erstreckt, **dadurch gekennzeichnet, dass** das längliche Well-artige Mittel (4) in Draufsicht eine "L"-förmige Ausführung zeigt.

3. Vorrichtung nach Anspruch 1 oder 2, welche ferner eine abgestufte Skalierung aufweist, die mit den Well-artigen Mitteln (3, 4; 3, 6) in Zusammenhang steht, wobei die Skalierung für die Zählung beliebiger mikrobieller Kolonien (7), die auf dem Kulturmedium (5) wachsen, ausgestaltet ist, und zwar durch Vergleichen der Position der Kolonien (7) mit Referenzmarkierungen der Skalierung.

4. Vorrichtung nach Anspruch 3, wobei die Skalierung ferner eine Referenzmarkierung aufweist, die in einer Entfernung von ungefähr 12,5 cm vom Ausgangspunkt der Strecke entfernt liegt.

5. Verfahren zur Zählung und Isolierung von Mikroorganismen in einer flüssigen Probe mit den Schritten:
- Eintauchen einer Öse in eine flüssige Probe, wodurch ein Anteil der Probe in der Öse aufgenommen wird;
- Ausstreichen des Anteils auf einem Kulturmedium (5), das in Well-artigen Mitteln (3, 4; 3, 6) einer Vorrichtung nach Anspruch 3 oder 4 enthalten ist,
**gekennzeichnet durch** die Schritte:
- Durchführung des Ausstreichens entlang einer nichtgeradlinigen Strecke, die sich kontinuierlich von einem Ausgangspunkt an einem Ende der Well-artigen Mittel (3, 4; 3, 6) bis zu einem Endpunkt der Well-artigen Mittel (3, 4; 3, 6) erstreckt;
- Wachsenlassen der ggf. in der Probe vorhandenen Mikroorganismen auf dem Kulturmedium (5), wodurch sich mikrobielle Kolonien (7) bilden, die voneinander getrennt sind;
- Zählung der Kolonien (7) **durch** Vergleichen der Positionen der Kolonien (7) auf dem Kulturmedium (5) mit den Referenzmarkierungen der abgestuften Skalierung, die mit den Well-artigen Mitteln (3, 4; 3, 6) in Zusammenhang steht.

6. Verwendung einer Vorrichtung nach einem der Ansprüche 1 bis 4 zur Isolierung und Zählung von Mikroorganismen.

## Revendications

1. Dispositif de comptage et d'isolement de micro-organismes dans un échantillon liquide, comprenant un récipient (2) muni de moyens de puits longitudinaux (3, 4) contenant un milieu de culture (5) sur lequel ledit échantillon peut être étalé le long d'un trajet qui s'étend en continu d'une position de départ située à une extrémité desdits moyens de puits (3, 4) jusqu'à une position terminale desdits moyens de puits (3, 4), ***caractérisé en ce que*** lesdits moyens de puits (4) montrent un tracé en forme de U dans une vue en plan.

2. Dispositif de comptage et d'isolement de micro-organismes dans un échantillon liquide, comprenant un récipient (2) muni de moyens de puits longitudinaux (3, 6) contenant un milieu de culture (5) sur lequel ledit échantillon peut être étalé le long d'un trajet qui s'étend en continu d'une position de départ située à une extrémité desdits moyens de puits (3, 6) jusqu'à une position terminale desdits moyens de puits (3, 6), ***caractérisé en ce que*** lesdits moyens de puits (4) montrent un tracé en forme de L dans une vue en plan.

3. Dispositif selon la revendication 1 ou 2, et comprenant de plus une échelle graduée associée auxdits moyens de puits (3, 4 ; 3, 6), ladite échelle étant agencée pour le comptage de toutes les colonies microbiennes (7) se développant sur ledit milieu de culture (5) par comparaison de la position desdites colonies (7) avec des repères de ladite échelle.

4. Dispositif selon la revendication 3, dans lequel ladite échelle comprend de plus un repère placé à une distance d'environ 12,5 cm d'un point de départ dudit trajet.

5. Procédé de comptage et d'isolement de micro-organismes dans un échantillon liquide, comprenant les étapes consistant à :
- plonger une anse dans un échantillon liquide pour ainsi piéger une partie dudit échantillon dans ladite anse ;
- étaler ladite partie sur un milieu de culture (5) contenu dans des moyens de puits (3, 4 ; 3, 6) d'un dispositif selon la revendication 3 ou 4 ;
***caractérisé par*** les étapes consistant à :
- effectuer ledit étalement le long d'un trajet non rectiligne qui s'étend en continu d'une position de départ située à une extrémité desdits moyens de puits (3, 4 ; 3, 6) jusqu'à une position terminale desdits moyens de puits (3, 4 ; 3, 6) ;
- permettre aux micro-organismes qui seraient présents dans ledit échantillon de se développer sur ledit milieu de culture (5) en formant des colonies microbiennes (7) qui sont isolées les unes des autres ;
- compter lesdites colonies (7) par comparaison de la position de celles-ci sur ledit milieu de culture (5) avec des repères d'une échelle graduée associée auxdits moyens de puits (3, 4 ; 3, 6).

6. Utilisation d'un dispositif selon l'une quelconque des revendications 1 à 4 pour l'isolement et le comptage de micro-organismes.
